# FASCICULE DE BREVET EUROPEEN

(11) **EP 1 357 857 B1**
(45) Date de publication et mention de la délivrance du brevet: **23.05.2007**
(21) Numéro de dépôt: 01994565.8
(22) Date de dépôt: 12.12.2001
(51) Int. Cl.: A61F 2/06

(54) **STENT A STRUCTURE TRESSEE TRIDIMENSIONELLE**
DREIDIMENSIONALER STENT MIT FLECHTSTRUKTUR
THREE-DIMENSIONAL BRAIDED STRUCTURE STENT

(30) Priorité: 12.12.2000 BE 200000783; 13.03.2001 EP 01870042
(43) Date de publication de la demande: 05.11.2003
(73) Titulaire: Cardiatis Société Anonyme, 7000 Mons (BE); F.R.I.D. R&D Benelux Sprl, 5032 Isnes (BE)
(72) Inventeur: FRID, Noureddine, B-1650 Beersel (BE)
(74) Mandataire: Van Straaten, Joop
(86) Numéro de dépôt international: PCT/BE2001/000210
(87) Numéro de publication internationale: WO 2002/047579

(56) Documents cités:
- EP-A- 0 804 909
- EP-A- 0 938 878
- WO-A-99/55256
- US-A- 5 957 974
- US-A- 6 004 346

## Description

L'invention concerne les endoprothèses luminales formées essentiellement d'une armature, sans recouvrement textile, généralement appelées « stents », et plus particulièrement les stents pour vaisseaux sanguins

L'implantation d'endoprothèses luminales est devenue au fil des ans une technique approuvée pour traiter les anévrismes, l'athérosclérose etc.

Cependant, un problème crucial n'a toujours pas été résolu à ce jour : la parfaite adéquation des caractéristiques mécaniques de ces endoprothèses et des organes dans lesquels elles sont implantées.

Même si un soin tout particulier a été apporté pour répondre à ces critères au moment de l'implantation, il se produit invariablement, à la longue, une disparité. Le corps humain subit en effet des altérations dues au vieillissement, tandis que l'endoprothèse présente un problème de stabilité dans le temps : rupture des filaments, altération de la structure, augmentation éventuel du diamètre( par relâchement de la structure ).

Les caractéristiques mécaniques d'un stent sont déterminées essentiellement par la structure de son armature. Quoiqu'il en existe de différents types, telles les armatures formées de tresses plates décrites dans WO-99/55256, l'armature à ce jour la plus adéquate est l'armature tressée cylindrique, telle que décrite notamment par Didcott dans GB-1205743, ou dans US 5 061 275.

Ce type d'armature se comprime aisément pour l'insertion, résiste bien à l'écrasement et conserve une souplesse relative compatible avec celle des vaisseaux sanguins ; la structure s'adapte au tracé sinueux d'artères rigides à traiter.

Jusqu'à présent, la recherche de l'armature optimale s'était orientée vers le choix du matériau, du pas de tressage, etc.

Ces recherches butent inévitablement sur une série de critères pratiques.

En adoptant un pas de tressage très faible (l'angle entre l'axe et les spires étant proche de 90°) ou en choisissant des fils épais, on accroît la force radiale (résistance à l'écrasement) mais on perd de la souplesse. Ce problème est encore plus crucial pour les stents et endoprothèses assemblés en plusieurs modules taillés au laser.

A l'inverse, un pas élevé, où l'angle formé entre l'axe et les spires avoisine par exemple 30° et l'usage de fils minces confèrent à l'armature une bonne souplesse mais une faible tenue à l'écrasement. En outre, un tel pas signifie un taux de raccourcissement important au moment de la mise en place de l'endoprothèse, ce qui entraîne un manque de précision lors du largage.

On a essayé, notamment dans EP-0 804 909, de combiner les fils métalliques avec des fibres textiles. Cette technique s'adresse uniquement au traitement des anévrismes et ne peut s'appliquer, par exemple, au traitement de la sténose carotidienne et même fémorale. Les résultats obtenus ne sont cependant pas probants :les filaments métalliques déforment la structure et créent le long de leur parcours hélicoïdal des dislocation et un écartement des fibres textiles. Les fibres sont sollicitées sous l'effet des pulsations provoquées par le flux sanguin et vont subir une érosion rapide par frottement contre les filaments métalliques (dont le module d'élasticité et le diamètre sont plus élevés).

Des résultats basés sur des études cliniques récentes ont montré que, dans le cas d'un anévrisme de l'aorte abdominale, 70 % de l'onde de pression est transmise à la paroi de la partie de l'anévrisme via l'endoprothèse. (Référence : *Communication at the 27^{th} Global Vascular Endovascular Issues Techniques Horizons*^{™} *nov-16-19,2000. Page V5.1).* Ces constatations ne sont pas surprenantes car l'hémodynamique nous enseigne que lorsque les parois sont minces, le travail nécessité pour le transport du sang s'accroît. On sait aussi que lorsque les vaisseaux sont trop gros, le volume de sang augmente au-delà de ce qui est nécessaire. Ces facteurs favorisent les anévrismes. Ceci montre qu'il faut mettre au point des structures plus stables et plus robustes. C'est le but que s'est fixé l'invention décrite ci-après.

L'objet de l'invention est un stent formé d'une armature tressée dépourvue de couche de revêtement dans laquelle l'armature comprend une pluralité de couches interconnectées entre elles, chacune de ces couches étant formée de deux nappes de fils métalliques, respectivement dextrogyre et lévogyre entrelacées entre elles, formant une trame, une pluralité de fils métalliques de chacune des couches étant intégrés dans la trame d'au moins une des couches adjacentes. Suite à l'étape de tressage, les fils métalliques du stent de l'invention subissent un traitement thermique leur imposant une transition de phase et un durcissement. L'armature comprend une pluralité de couches assurant à la paroi latérale de ce stent une porosité transformant un flux de convection hémodynamique au travers de cette paroi en flux de diffusion.

Le stent multicouche présente des avantages par rapport aux autres stents tressés, monocouches ; il bénéficie d'une force radiale accrue, d'une tenue dans le temps allongé, d'une meilleure adaptation due au nombre de couches au type de l'artère et de sa pathologie.

La structure fait appel à un seul type de matériau ce qui assure sa robustesse et son homogénéité.

La structure donne, aussi, la possibilité de coller une couverture en PTFE ou en Dacron.

Dans le cas d'une armature métallique, les fils sont de préférence choisis parmi les matériaux suivants [Phynox®, Elgiloy®, titane et alliages ou titane Nitinol]. Le traitement thermique (qui donne aux fils la stabilité structurelle et la rigidité nécessaire) est postérieur au tressage, et ce au contraire des stents hybrides de EP 0 804 909 A2 cités plus haut.

Il est en effet communément admis que tout fil durci par traitement thermique perd de son élasticité et de sa déformation plastique et que par conséquent il devient rigide. Il est donc fortement déconseillé de re-travailler un fil durci par traitement thermique, comme c'est le cas dans un stent hybride : le traitement thermique est impossible à appliquer a posteriori, en présence de fibres textiles, qui fondraient ou brûleraient. Suivant une forme de réalisation avantageuse, l'armature comprend des fils d'épaisseurs différentes.

L'épaisseur des fils peut s'étager sur une gamme comprise entre 25 et 60 microns.

L'avantage de l'utilisation de fils minces avec une taille des interstices (comparables aux pores d'une structure filtrante) structurés en multicouches, ajustés à une dimension entre 100 et 200µm est qu'ils perturbent peu le flux sanguin : il est donc possible de les utiliser pour des endoprothèses sans couches de revêtement (stents), qui empêchent les particules venant du réseau vasculaire de remonter vers le cerveau et de provoquer des thromboses voire des attaques cérébrales.

L'avantage de l'utilisation de fils plus épais est qu'ils assurent une meilleure tenue contre la paroi des vaisseaux et permettent à l'endoprothèse d'encaisser sans dommages les diverses sollicitations auxquels les vaisseaux sont soumis, notamment au niveau du cou et du genou.

L'utilisation de couches interconnectées résout également la corrélation épineuse de trois problèmes préoccupants que ne résout pas la technique antérieure : en utilisant des nappes de fils avec des matériaux de caractéristiques mécaniques différentes ou aussi des structures faites en modules assemblés, on constate que les endoprothèses de l'art antérieur (principalement celles destinées aux anévrismes), ont tendance à migrer longitudinalement, à changer de forme dans le temps et à se dégrader.

A l'inverse, dans une prothèse suivant l'invention, les propriétés mécaniques des fils des différentes nappes peuvent être équilibrées de façon à se compenser parfaitement et d'assurer une stabilité à long terme.

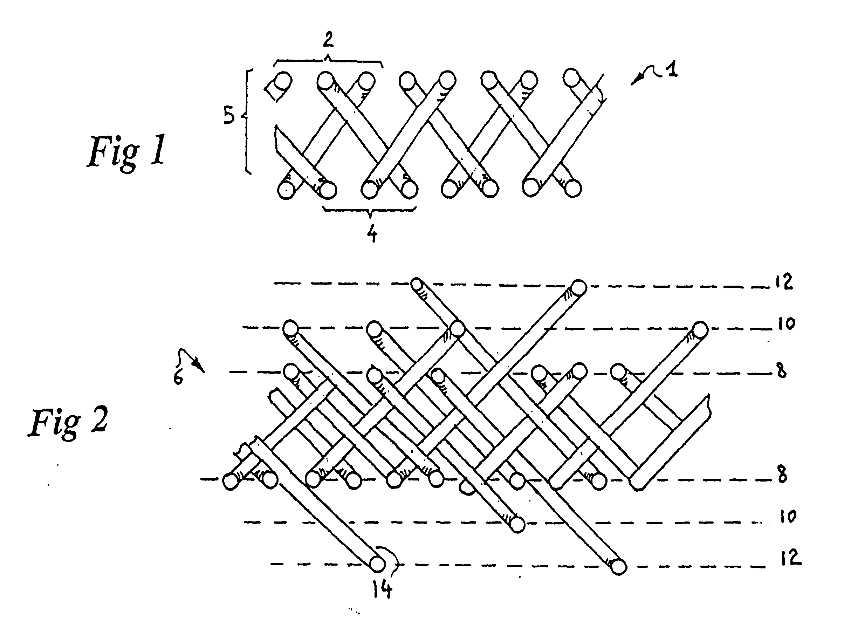

D'autres particularités et avantages de l'invention ressortiront de la description ci-après de modes de réalisation particuliers de l'invention, référence étant faite aux dessins annexés dans lesquels
La Fig. 1 est une vue latérale d'une armature de stent tressé classique;
La Fig. 2 est une vue schématique simplifiée de l'armature de stent de l'invention.
Les Fig. 4 et 5 montrent la réduction d'un anévrisme avec le stent de l'invention.

L'armature tressée classique 1 est formée d'une tresse simple, deux nappes 2, 4 de fils, respectivement dextrogyre 2 et lévogyre 4 s'entrecroisent, formant une trame 2, 4 simple.

L'armature 6 de l'invention est une tresse multiple qui comprend, dans l'exemple illustré, trois couches 8, 10, 12 dont les nappes ne sont pas distinctes : au moment du tressage, un certain nombre de fils 14 des nappes de la première couche 8 sont entrelacés avec les nappes de la deuxième couche 10 et/ou de la troisième couche 12, formant une trame complexe( ceci vaut pour la figure représentée, mais il va de soi que l'entrelacement peut se poursuivre jusqu'à une Nième couche si le nombre de couches est N). Cette façon de procéder ouvre des possibilités énormes pour ajuster les caractéristiques de l'armature. Non seulement elle rend possible une grande variété de "standards" en fonction des organes visés, mais encore, elle permet pratiquement un ajustement au cas par cas en jouant sur le pas du tressage, le diamètre et la nature des fils 14, la densité du tressage, le nombre des couches 8, 10, 12 , le nombre de fils 14 de différents diamètres et l'entrelacement des couches.

Il serait bien sûr inutile d'évoquer tous ces avantages si les endoprothèses munies d'une telle armature ne pouvaient pas être mises en place avec les équipements existants.

Or, c'est un des aspects inattendus de l'invention, on constate qu'en dépit du grand nombre de fils 14 utilisés, de l'épaisseur des couches 8, 10, 12 successives, la complexité de la structure, la tresse exclusivement métallique multiple peut être très aisément réduite jusqu'à un diamètre comparable à celui d'une armature classique 1 : à l'inverse des fils ou des brins des armatures multicouches composites ou même des monocouches , ceux du présent multicouche ont tendance à occuper l'espace d'une façon plus rationnelle, vraisemblablement grâce à l'interpénétration complexe des couches entre elle.

Il est donc possible d'utiliser sans difficulté un introducteur classique pour la mise en place d'un stent équipé de la nouvelle structure, même dans des vaisseaux de diamètre réduit.

Par ailleurs, le stent de l'invention peut assumer, après déploiement, de très grands diamètres (notamment pour une dissection aortique ou dans le cas de l'oesophage) sans risque d'écrasement.

La présente structure permet également aux couches 8, 10, 12 constituées de fils 14 de différents diamètres d'agir en synergie. Des essais cliniques menés par des praticiens (chirurgiens, radiologues et cardiologues) où l'on se contente d'introduire l'un dans l'autre deux stents 1 de caractéristiques différentes se sont soldés par des résultats mitigés ou par des échecs, alors que la présente structure 6 accroît notablement la résistance à l'écrasement sans réduire la flexibilité.

Cette caractéristique est importante notamment dans le cas de traitement d'anévrismes. En effet, un anévrisme montre au fil du temps une tendance à se raccourcir dans le sens longitudinal. Un stent classique placé dans ces circonstances aura tendance à onduler et finalement à s'écraser, ce qui n'est pas le cas de la présente structure.

Par ailleurs, comme il a été précisé plus haut, la structure en couche multiple 6 permet l'utilisation de fils de très fin diamètre pouvant jouer le rôle d'une structure filtrante en combinaison avec des fils de renfort plus épais.

L'entrelacement de ces fils assure une répartition régulière dans l'espace, assurant un maillage régulier propice à un filtrage uniforme des particules.

Outre leurs caractéristiques mécaniques propres, on peut également utiliser avec avantages les particularités confiées aux fils par un traitement technique adéquat.

On peut ainsi tirer parti de l'usage de fils en Nitinol tels que décrits dans la demande PCT/BE 98/00076 pour obtenir un raffermissement de la structure après sa mise en place.

Le recours à la structure multicouche et à des fils métalliques dont le diamètre se situe entre 25 µm et 60µm permet la réalisation d'une structure filtrante à la fois stable et efficace.

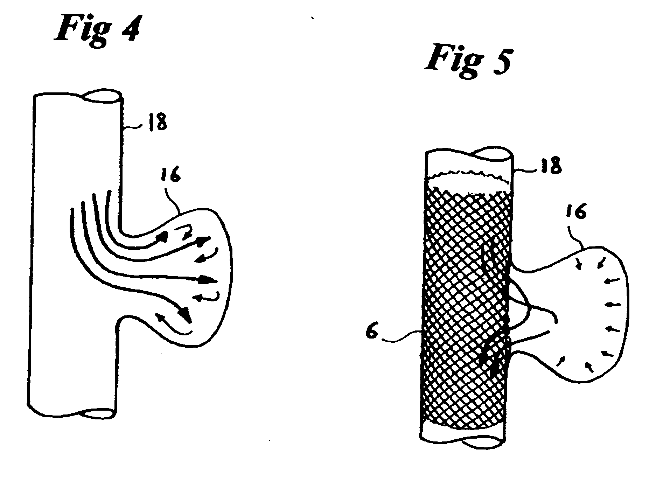

Les Fig. 4 et 5 illustrent la possibilité qu'offre le stent de l'invention de résoudre d'une façon à ce jour peu conventionnelle le problème causé par les anévrismes.

La démarche désormais classique de réduction des anévrismes 16 consiste à placer dans le vaisseau 18 atteint une endoprothèse munie d'une couverture étanche en polymère. La déformation pratiquement inévitable de cette endoprothèse conduit cependant à l'apparition progressive de fuites entre cette endoprothèse et la paroi du vaisseau 18, particulièrement dans le cas d'anévrismes 16 fusiformes. La poche formée par l'anévrisme 16 est donc soumise aux mêmes sollicitations qu'auparavant et elle cesse de se résorber. Il est cependant possible de traiter des anévrismes sans faire appel à des couvertures dites étanches. Les études (Annals of Biomedical Engineering.Vol.25 pp .460-469; 1997) montrent qu'on mettant un stent dont les parois sont dans une fourchette de porosité bien précise on peut théoriquement altérer l'hémodynamique dans un anévrisme en transformant le flux de convection (comme montré à la Fig 4) en flux de diffusion (voir Fig 5), ce qui diminue la pression dans la poche 16, laquelle peut se résorber normalement. En ajustant le nombre de fils, le nombre de couches et la taille des intervalles entre les fils, il est possible d'obtenir la porosité requise avec le présent stent, ouvrant ainsi une possibilité pratique d'appliquer la technique décrite plus haut.

## Revendications

1. Endoprothèse luminale formée d'une armature tressée (6) dépourvue de couche de revêtement comprenant une pluralité de couches (8, 10, 12)de fils biocompatibles métalliques entrelacées entre elles, chacune de ces couches (8, 10, 12) étant formée de deux nappes de fils 14, respectivement dextrogyre et lévogyre entrelacées entre elles, formant une trame (5), une pluralité de fils (14) de chacune des couches (8, 10, 12) est intégrés dans la trame d'au moins une des couches adjacentes (8, 10, 12), **caractérisée en ce que** la dite armature a été soumise à un traitement thermique lui imposant une transition de phase et un durcissement par traitement thermique après avoir été tressée, la paroi latérale de cette endoprothèse ayant une porosité transformant, lorsque elle est placée dans un vaisseau atteint d'un anévrisme, par diminution de la pression dans l'anévrisme,un flux de convection hémodynamique au-travers de cette paroi en flux de diffusion.

2. Endoprothèse luminale suivant la revendication 1 **caractérisée en ce que** le matériau des fils (14) est choisi parmi les matériaux suivants [acier inoxydable, Phynox®, Elgiloy®, titane et ses alliages, Nitinol].

3. Endoprothèse luminale suivant l'une quelconque des revendications précédentes **caractérisée en ce que** l'armature (6) comprend des fils métalliques (14) d'épaisseurs différentes.

4. Endoprothèse luminale suivant la revendication 3 **caractérisée en ce que** l'armature (6) comprend des couches formées de fils (14) d'épaisseur différente.

5. Endoprothèse luminale suivant l'une quelconque des revendications précédentes **caractérisée en ce que** l'armature comprend des fils métalliques (14) de natures différentes.

6. Endoprothèse luminale suivant l'une quelconque des revendications 3, 4, 5 **caractérisée en ce que** l'épaisseur des fils (14) s'étage au moins sur une gamme comprise entre 25 et 60 micromètres.

## Claims

1. Luminal endoprosthesis formed of a braided framework (6) devoid of covering, comprising a plurality of layers (8, 10, 12) of biocompatible metal wires which are interlaced with each other, each of these layers (8, 10, 12) being formed by two plies of wires (14), which are respectively dextrogyratory and laevogyratory, and which are interlaced and form a lattice (5), a plurality of wires (14) of a given layer being integrated in the lattice of at least one of the adjacent layers (8, 10, 12) **characterised in that** said framework has been subjected to a thermical treatment imparting to it a phase transition and a hardening after having been braided, the lateral wall of said endoprothesis having a porosity transforming, when it is placed in a vessel bearing an aneurysm, by reducing the pressure in said aneurysm, a haemodynamic convection flow through this wall to a diffusion flow.

2. Luminal endoprosthesis according to claim 1, **characterised in that** the material of the wires is chosen from among the following materials [stainless steel, Phynox®, Elgiloy®, titanium and its alloys, Nitinol].

3. Luminal endoprosthesis according to any one of the preceeding claims, **characterised in that** the framework (6) comprises metal wires (14) of different thicknesses.

4. Luminal endoprosthesis according to claim 3, **characterised in that** the framework (6) comprises layers formed by wires (14) of different thickness.

5. Luminal endoprosthesis according to any one of the preceeding claims, **characterised in that** the framework comprises metal wires of different types.

6. Luminal endoprosthesis according to any one of the preceeding claims, **characterised in that** the thickness of the wires lies at least within a range of between 25 and 60 micrometres.

## Patentansprüche

1. Luminale Endoprothese, die aus einer geflochtenen Armierung (6) ohne Verkleidungsschicht besteht, die mehrere Schichten (8, 10, 12) von biokompatiblen Metalldrähten umfasst, die untereinander verflochten sind, wobei jede dieser Schichten (8, 10, 12) aus zwei Decken von rechtsdrehenden beziehungsweise linksdrehenden Drähten 14 gebildet ist, die untereinander verflochten sind, die einen Schuss (5) bilden, wobei mehrere Drähte (14) von jeder der Schichten (8, 10, 12) in den Schuss von mindestens einer der benachbarten Schichten (8, 10, 12) integriert ist, **dadurch gekennzeichnet, dass** die Armierung einer Wärmebehandlung unterzogen wurde, die ihr einen Phasenübergang und eine Härtung durch Wärmebehandlung auferlegt hat, nachdem sie geflochten wurde, wobei die Seitenwand dieser Endoprothese eine Porosität hat, die, wenn sie in ein von einem Aneurysma befallenen Gefäß eingesetzt wurde, durch Verminderung des Drucks im Aneurysma einen hämodynamischen Konvexionsstrom durch diese Wand in einen Diffusionsstrom umwandelt.

2. Luminale Endoprothese nach Anspruch 1, **dadurch gekennzeichnet, dass** das Material der Drähte (14) unter den folgenden Materialien gewählt wird [rostfreier Stahl, Phynox®, Elgiloy®, Titan und seine Legierungen, Nitinol].

3. Luminale Endoprothese nach einem der vorhergehenden Ansprüche, **dadurch gekennzeichnet, dass** die Armierung (6) Metalldrähte (14) unterschiedlicher Dicke umfasst.

4. Luminale Endoprothese nach Anspruch 3, **dadurch gekennzeichnet, dass** die Armierung (6) Schichten umfasst, die aus Drähten (14) unterschiedlicher Dicke gebildet sind.

5. Luminale Endoprothese nach einem der vorhergehenden Ansprüche, **dadurch gekennzeichnet, dass** die Armierung Metalldrähte (14) unterschiedlicher Natur umfasst.

6. Luminale Endoprothese nach einem der Ansprüche 3, 4, 5, **dadurch gekennzeichnet, dass** die Dicke der Drähte (14) stufenförmig mindestens in einem Bereich zwischen 25 und 60 Mikrometern ansteigt.
